# EUROPEAN PATENT APPLICATION

(11) **EP 1 384 446 A1**
(43) Date of publication of application: **28.01.2004**
(21) Application number: 02017016.3
(22) Date of filing: 27.07.2002
(51) Int. Cl.: A61B 18/20

(54) **Hand-held laser for skin treatment**

(71) Applicant: Lux Medico ApS, 8000 Aarhus C (DK)
(72) Inventor: Pedersen, Joergen Ejler, 5000 Odense C (DK)
(74) Representative: Gregersen, Niels Henrik

(57) **Abstract**

An apparatus for skin treatment comprising a casing of a size which is easily handheld, a laser system inside said casing including a laser with an electronic circuit, said laser arranged such that produced laser radiation from said laser exits said casing through an exit system of said casing, wherein the exit system comprises a safety device that only allows firing of the laser when said exit system is touching the skin to be treated and the direction of said laser radiation is directed onto the skin.

## Description

### FIELD OF THE INVENTION

The present invention relates to a hand-held laser apparatus, preferably for depilation.

### BACKGROUND OF THE INVENTION

In order to permanently remove hair, it is well-known to damage the hair roots by laser radiation. In US patent no. 5 632 741 by Zavislan and Thomson, a depilation system is disclosed, where a hand piece is used for directing a laser beam into the root structure of a hair. The hand piece is connected to a large CO₂ laser generating pulses with pulse energy of 25-250 mJ thereby achieving a temperature of more than 100° C in the root of the hair.

Laser depilation apparatuses according to prior art are large and expensive and designed for use by physicians and not suitable for private use in the user's home. For example, the price for typical laser epilators starts at 50,000 US-dollars. It would be desirable to have a depilation apparatus, which is smaller and more affordable than the large systems described above. In this case, however, it has to be assured, that the system is safe even for users without a thorough education in using laser radiation, because the laser radiation necessary to remove hair has an energy and a wavelength that may cause damage, if directed into an eye or onto dark skin.

Small hand-held lasers are generally known, for example as disclosed in US patent no. 5 697 700 by Huang for use as a handy pointer. A laser with a higher power is disclosed in US patent no. 6 370 173 by Martin, wherein the laser has a heat sink and a ventilator in order to dissipate the produced heat by the laser diode with one to sixty watts or more. The ventilation system and the rather high power of the system result in a heavy, not very handy device. For low level laser treatment (LLLT), especially for tissue healing at a tissue depth of several centimeters, a hand-held laser device is known from US patent no. 5 464 436 by Chadwick. Other hand-held lasers for acupuncture are commercially available from numerous companies, for example the Danish company Unilaser®. These lasers - not designed for hair removal - may accidentally be fired in directions other than the skin to be treated, which implies a high risk of injury to the user and bystanders, for example when laser light is directed towards the eye of a person either directly or via a reflecting surface. In addition, strong absorption of laser radiation in the skin may cause skin burns. Dark skin absorbs laser light more effectively than fair skin and it is, thus, desirable that the energy of the laser radiation is adjusted according to the pigmentation of the skin to be treated.

### DESCRIPTION OF THE INVENTION

It is the purpose of the invention to provide a handy laser apparatus for skin treatment that is easily affordable by private users and safe in handling.

This purpose is achieved by an apparatus for laser treatment comprising a casing of a size which is easily hand-held, a laser system inside the casing including a laser with an electronic circuit, the laser arranged such that produced laser radiation from the laser exits the casing through an exit system of the casing, and a connection to an electrical power supply inside, or external to, the casing to provide power to the apparatus, wherein the exit system comprises a safety device that only allows firing of the laser when the laser radiation is directed onto the surface of the subject to be treated. The subject to be treated is primarily human skin, but the use of the invention is general and not limited in any respect.

Typical laser radiation for removal of hair is in the near-infrared or infrared region. For example, such an apparatus can be provided with a diode laser system involving a diode that emits laser radiation at a wavelength of 800 nm. The wavelength region of between 600 and 1100 nm is advantageous in that the laser radiation is transmitted through several mm skin without substantial heating of the skin. However, the roots of hairs or the hairs themselves absorb the laser radiation resulting in heating of the roots. to the extend that the hairs fall out and further hair growth is prevented. The dermatological effect may be removal of hairs from the skin, which requires a temperature of the root of the hair of at least 60°C-70°C. In order to achieve this temperature, a laser power of 80 mW has proved to be sufficient.

Possible applicable lasers are rubin laser (ca. 690 nm), alexandrite laser (ca. 780 nm), titanium sapphire laser (ca. 800 nm), and neodymium YAG laser, ND:YVO at 1064 nm.

Alternative dermatological treatment may be removal of acne, tattoos, benign pigmented lesions (for example birthmarks), microvascular malformations (for example port wine stains), vascular lesions (for example spider veins) or wrinkles. In these cases, it is not always necessary to have a wavelength in the near infrared. Essential is the absorption of the laser radiation at the point of material damage or material removal. For example, a black tattoo may be removed with any colour of laser radiation, whereas blue and green tattoo colours may best be treated with red laser light. For removal of acne, blue lasers are applicable, for example by blue diode lasers or frequency doubled infrared lasers. For removal of Spider veins, yellow or red lasers are suitable.

Laser radiation with such wavelengths and necessary intensity is dangerous if directed into the eye of a person or applied to dark skin. Therefore, the apparatus according to the invention foresees a safety device such that it is assured that the laser radiation is directed onto the skin.

The safety device may comprise at least one from the following
- a mechanical pressure switch arranged to be activated when the exit system of the apparatus is pressed against the surface of the subject to be treated, the pressing being in a direction which requires an orientation of the apparatus such that the laser radiation is directed towards the surface of the subject to be treated;
- a pressure sensor coupled to a safety switch only allowing the laser to fire when the pressure exerted on the pressure sensor is higher than a predetermined level, the pressure being in a direction substantially normal to the surface of the subject to be treated;
- a temperature sensor, for example a thermistor, coupled to a safety switch only allowing the laser to fire when the temperature at the surface of the subject to be treated as measured with the temperature sensor is within a predetermined range of temperatures, for example 27°C - 40°C in case of skin treatment;
- a pH sensor coupled to a safety switch only allowing the laser to fire when the pH at the surface of the subject to be treated as measured with the pH sensor is within a predetermined range of pH, for example between a pH of 4.5 and a pH of 5.5;
- a resistivity sensor coupled to a safety switch only allowing the laser to fire when the resistivity at the surface of the subject to be treated as measured with the resistivity sensor is within a predetermined range of resistivity, for example between 10 kΩ/mm and 1 MΩ/mm;
- a conductivity sensor coupled to a safety switch only allowing the laser to fire when the conductivity at the surface of the subject to be treated as measured with the conductivity sensor is within a predetermined range of conductivity;
- a radiation detector configured to receive reflected laser radiation, said radiation detector being functionally coupled to a safety switch stopping said laser to fire, for example for a certain time interval or until the next time the laser is switched on, when the intensity of said reflected laser radiation detected by said radiation detector is outside a predetermined range of intensity,
- an additional radiation source, preferably a light emitting diode or a low intensity laser (for example 1 mW), and a radiation detector for detection of radiation from said additional radiation source reflected on a surface to be treated, said radiation detector being functionally coupled to a safety switch only allowing said laser to fire when the intensity of said reflected radiation from said additional radiation is within a predetermined range of intensity.
- a radiation detector receiving reflected laser radiation and an evaluation device, preferably an evaluation computer, functionally connected to said radiation detector for determining the type of surface reflecting the laser radiation,
- an additional radiation source, preferably a light emitting diode, and a radiation detector for detection of radiation from said additional radiation source reflected on a surface to be treated, said radiation detector being functionally connected to an evaluation device, preferably an evaluation computer, for determining the type of surface reflecting the laser radiation.

The evaluation device may be used for determining, whether the laser should be able to fire or not. In a further embodiment of the invention, the evaluation device is functionally connected to an intensity adjustment device adjusting the intensity of the laser radiation according to the intensity of the light reflected from the surface. As dark skin may be damaged more easily than light skin during laser irradiation due to the reduced reflectivity of radiation, an intensity adjustment device is increasing the safety of the device during use, as the intensity of the laser radiation is adjusted according to the pigmentation of the skin to be treated. In practice, the apparatus is configured to treat the intensity of the reflected radiation as being indicative of the degree of pigmentation of human skin and the intensity of said laser radiation is automatically adjustable in relation to a predetermined relationship between degree of skin pigmentation and intensity of laser radiation. Such a predetermined relationship may be stored as a table or calculated during use in accordance with a mathematical model, for example an analytical mathematical expression.

In order to achieve a well-defined conductivity, resistivity, pH or reflectivity on the surface of the subject to be treated, preferably skin, the surface may be exposed to a certain liquid, for example sprayed on the surface or applied to the surface by a dispensing pen. This way, the certain liquid acts as an additional safety factor, because application of the liquid is necessary for laser irradiation. Such a liquid may be coloured in order to mark the region to be illuminated. The liquid may also be provided with substances that have a cooling effect, which may be felt as pleasant by the user. Further dermatologically advantageous ingredients may be contained in such a liquid for optimum convenience for the user.

For example such a liquid may be used in a kit laser treatment comprising an apparatus according any one of the preceding claims and a liquid for application to the surface of the object to be treated, wherein said liquid has at least one from the group consisting of a preselected marking colour, conductivity, resistivity, pH or reflectivity, or a cooling effect.

Preferably, the exit system is configured to create a closed system in combination with the surface of the object to be treated, such that no laser light can escape uncontrolled during use of the apparatus. The apparatus may be constructed such that the laser can only fire when the exit system touches the surface of the object to be treated, for example the skin. By arranging sensors in the most forward part of the apparatus, for example at the proximal end of a tube-like system, this part has to touch the surface in order for the laser to fire. At the same time, this forward part closes the volume around the surface region to be treated and no laser light can escape uncontrolled from this volume, This reduces the risk for accidents.

The apparatus according to the invention may be powered through a cord by an external power supply, for example connected to the public electricity system. However, for simplicity, the power supply is preferably a battery system inside the casing.

The laser may be a laser diode, but the light source is not restricted to laser diodes. The laser system may comprise a single laser diode resulting in a single laser spot or a laser diode array for creating a line of radiation, which on the one hand is easier to handle for the user and on the other hand involves a faster result due to the larger spot. In case a laser source with a spot-like, for example circular, beam profile is used, a line focus may be obtained by focusing with for example a cylindrical lens, anamorphic prisms or diffractive optics.

In case the laser is mounted close to the surface of the subject to be treated, the direct spot from the laser may be used for the dermatological treatment. However, it is preferred that the depth of the region to be treated can be set prior to the treatment, because different kinds of dermatological treatment may require different depths. Therefore, it is preferred that a focusing lens is arranged in front of the laser, typically in the exit system. Using a focusing lens has an intrinsic security aspect. As the laser radiation is largely divergent after the focus near the exit region, the light power per area decreases as the square of distance from the focus. This implies that already after a relatively short distance from the apparatus, the danger from the laser radiation is decreased substantially.

For hair removal, the focus of the laser may be adjusted to 0.5 - 5 mm below the skin surface. If the laser treatment is used for hair of the beard, the required depth may be as large as 5 mm, though typically the adjusted focus depth in the skin is 1-4 mm, preferably 3 mm. However, the depth of the focus may be chosen in dependence of the wavelength, because the effect of scattering in the skin leading to broadening of the laser beam is dependent on the wavelength.

In a further embodiment of the invention, the focusing lens - or focusing lens system-may be adjustable such that the focus can be varied within a range of depth in the subject to be treated. Alternatively, the focusing lens system may be exchangeable, eventually in combination with an exchangeable exit system.

The apparatus according to the invention may for some embodiments cover the illuminated spot such that the user is not able to see the treated region clearly. In order to take this aspect into account, the invention in a further embodiment foresees a magnifying lens attached to the casing and directed towards the surface spot for laser treatment. The magnifying lens may be coated with a film or dyed with a colour such as to prevent or strongly attenuate the transmission of reflected laser light.

It is important that the strong laser light is not accidentally directed into eyes, which as a result may be damaged leading to blindness. Therefore an extra security feature may be incorporated. This security feature being an additional switch being arranged resiliently with counter force such that the switch may be pressed but not locked in the pressed state, wherein the laser only can fire when the additional switch is pressed. Thus, the user has to press the finger on the switch in order to use the apparatus according to the invention. As soon as the finger is released, the switch returns by the resilient force back to the original position where no laser irradiation is possible.

In order to signal the powered state of the apparatus, it may have a main switch for the main power supply and a main control lamp, which lights up when the main switch is turned on.

Apart from the main control lamp, which indicates the general power state, the apparatus may comprise a laser control lamp, which is illuminated when the laser is firing. For example, the laser control lamp may be a Teflon tip in the exit system, the Teflon tip confining the laser radiation near the treatment spot on the surface. Thus, the tip in the exit system is illuminated by the laser radiation and indicates clearly to the user, that the laser is firing and also where the illuminated spot is.

When not in use, the laser may be protected against dust and dirt by a cover. By covering the exit system laser radiation is prevented from exiting the exit system, which is a further safety factor. Preferably, the cover is locked to the apparatus with a locking mechanism, such that the cover is only demountable after unlocking of the locking mechanism. Such a locking system may advantageously comprise an alphanumerical code lock.

For highest safety reasons, the user may be equipped with protecting glasses for filtering the high intensity radiation in order to protect the eyes. Also, in order not to be able to direct reflected laser radiation into the eyes of other persons, the apparatus according to the invention may be used solely in closed rooms. In case that a user enters the room, the apparatus may be equipped with a receiver for receiving an alarm signal that has been sent from a sensor that has recognised the opening of a door in the room. As soon as the apparatus according to the invention receives the alarm signal, the laser is turned off, until a safe environment has been achieved again.

It has turned out for some cases that especially blond or red hair may contain too little pigment in order to be removed by the laser irradiation from an apparatus according to the invention. In this case, the hair may be toned with a toner substance as is known from hairdressing activities, where different colours are applied to the hair.

### SHORT DESCRIPTION OF THE DRAWINGS

The invention will be explained in more detail with reference to the drawing, where
FIG. 1 shows an apparatus according to the invention,
FIG. 2 shows an alternative embodiment of an apparatus according to the invention,
FIG. 3 shows a diagram of a safety circuit.

### DETAILED DESCRIPTION / PREFERRED EMBODIMENT

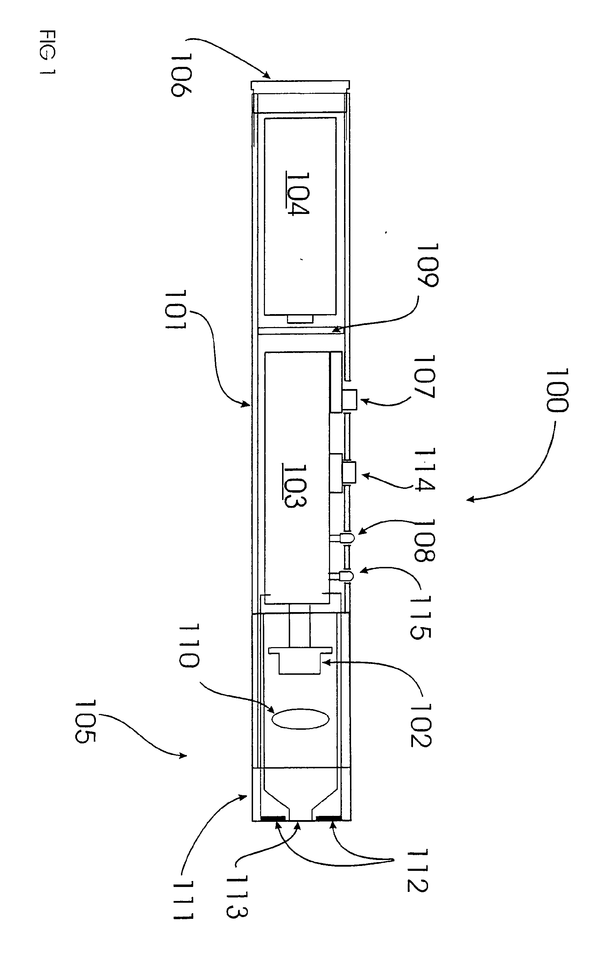

In FIG. 1, an apparatus 100 according to the invention is shown. It comprises a casing 101 of a size that is easily hand-held. Such a size may typically be between 1 cm and 5 cm in diameter and with a typical length of between 5 cm and 30 cm. The casing 101 contains a diode laser system including a laser diode 102 with an electronic circuit 103. As an electrical power supply to provide power to the apparatus, a battery 104, for example rechargeable, is enclosed in the casing 101 and fixed by a fixing arrangement 109. Alternatively, the power may be supplied from an external power supply connected to the apparatus 100 through an electrical cord.

The casing 101 is closed at the back end by a rear cover 106 that is screwed into the casing 101. In order to activate the laser, a main switch 107 has to be activated. When activated, a power indicator 108, for example a light emitting diode, indicates that the power has been switched on.

The laser 102 is arranged such inside the casing 101 that produced laser radiation from the laser 102 exits the casing 101 through an exit system 105. A laser used in the apparatus can be a continuously firing diode laser as well as a pulsed laser. However, the former is preferred, as it only requires a relatively simple electronic circuit 103.

The exit system comprises an optical lens system 110 for focusing the laser radiation from the laser 102. After being focused by the lens system 110, the laser radiation traverses a safety tube system 111. The safety tube system 111 is coupled to a conductivity switch 112, which is activated when the exit system 105 is pressed against the surface of the subject to be treated. Activation of the conductivity switch 112 results in powering of the laser system such that the laser 102 is only able to emit radiation when the exit system 105 is touching the surface of the subject to be treated, where both sensor pads of the conductivity switch 112, preferably situated on each side of the laser beam exit aperture 113, have to simultaneously touch the surface of the subject to be treated thus assuring an orientation of the apparatus 100 such that the laser radiation is directed towards the surface, for example normal to the surface.

As an alternative to the conductivity switch 112, the safety tube system 111 may be used in connection with a temperature sensor, a pH sensor, a mechanical pressure switch, a pressure sensor, or a resistivity sensor coupled to a safety switch only allowing the laser to fire when the measurements by the sensors are within a predetermined range and the direction of the laser beam is substantially normal to the surface of the subject to be treated. The sensors may be used in combination as well, depending on the kind of safety that is preferred in the actual treatment case. In order to provide the laser 102 and the electronics 103 with power, an additional switch 114 has to be activated, which is an additional safety aspect for preventing injuring mistakes during usage of the apparatus. Such an additional switch 114 may be arranged resiliently with counter force such that the switch 114 may be pressed but not locked in the pressed state. This switch assures that the user indeed has to deliberately activate the apparatus for firing the laser.

Once the measurements with the sensor or sensors are within the predetermined range, and/or the mechanical additional pressure switch 114 is activated, the laser may fire. Since the laser radiation is entirely encapsulated by the safety tube 111, the user is unable to see the radiation. Therefore a further light emitting diode 115 or other indicator is used to indicate when the laser 102 is actually firing.

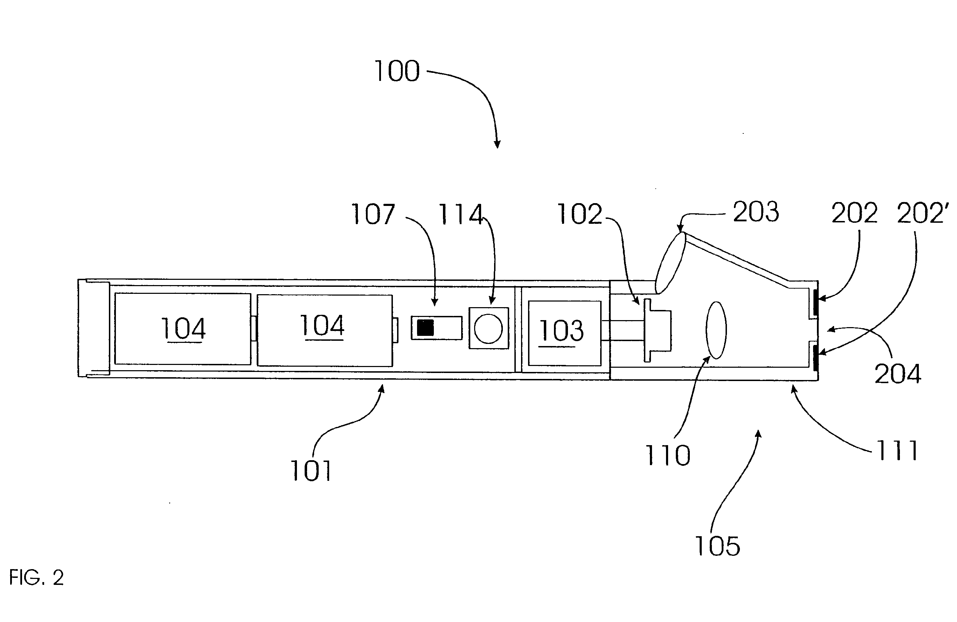

An alternative embodiment is shown in FIG. 2. The apparatus 100 comprises two batteries 104 inside the casing 101 and a non-latching switch 114 in addition to a main switch 107 for power supply.

The lens system 110 may be constructed adjustable in order to be able to vary the distance from the exit system 105 to the focus of the laser radiation inside the skin of a patient, for example at a depth of 0.5 mm to 5 mm.

In the shown embodiment, the exit system 105 is equipped with at least two sensors 202, 202', which may be pressure sensors, temperature sensors, pH sensors, conductivity sensors, or resistivity sensors or a combination thereof. Having at least two sensors 202, 202' to touch the surface of the subject to be treated assures that the apparatus 100 according to the invention is held in an orientation which is to a high degree normal to the surface, for example the skin of the patient. The latter assures that the laser radiation is indeed directed onto the surface or into the subject, for example the skin, to be treated.

The apparatus 100 may advantageously comprise a magnifying lens 203 attached to the casing 102 and directed towards the surface spot 204 for laser treatment. Through a magnifying lens 203 in the exit system 105, the user may observe the treatment spot 204. This facilitates the use of the apparatus 100, because the user in a more safe and convenient way may find the spot 204 on the surface of the skin, where a radiation treatment shall be applied, for example the removal of hair.

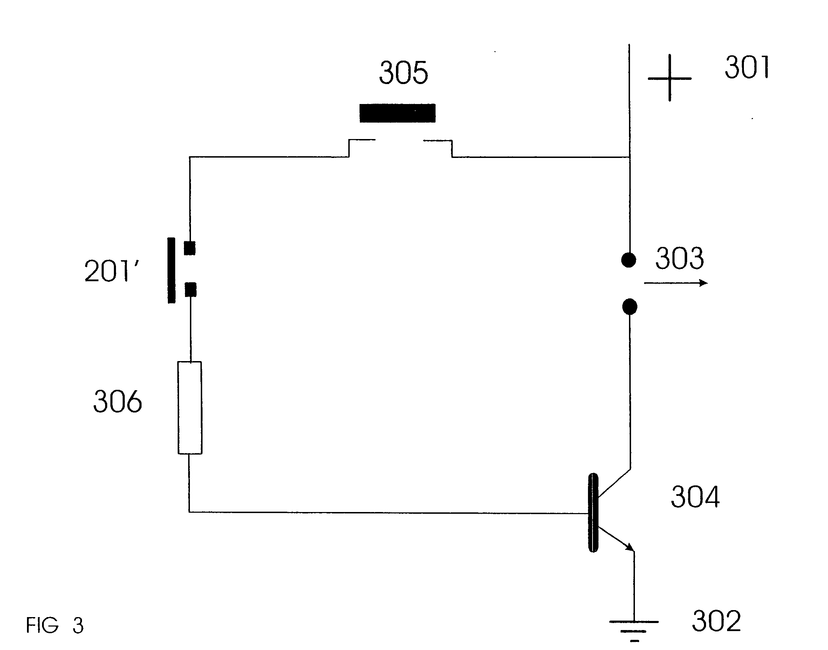

In FIG. 3, a diagram is illustrated for a safety arrangement. Between the two battery poles 301, 302, electrical power can be extracted - which is indicated with an arrow 303 - for the electronics 103 and the laser 102. However, this is only possible, if the transistor 304, for example a Darlington transistor, is open for current through the collector. In order to open for the collector current, a small emitter current has to flow, which is achieved when the additional switch 201' is closed while, simultaneously, the sensor 305 measures a parameter within a predetermined range. An emitter current of 1 microamp may trigger a collector current of 1 amp for the laser 102 and the electronics 103. A resistor 306 is preferably used for stability reasons, though this is not strictly necessary.

The exit system 105 of the apparatus according to the invention may be equipped with a detector that detects reflected light from the surface at which the laser radiation is directed. Such a detector with corresponding electronics may be included in the safety system of the apparatus. For example, the detector may determine, whether the surface may indeed be the skin of a person. Thus, the reflected radiation has to be within a predetermined range for the laser to continue firing. For example, if there is no reflection at all, the laser is not pointing at a nearby reflective surface or not on a reflective surface with a convenient angle and the laser may be stopped.

By detecting the radiation that is reflected from the skin of the treated person, the type of skin may be determined. A high reflectivity may indicate a skin of light colour, while a low reflection may indicate a darker tan in the skin. A darker skin will typically absorb more radiation than light skin and is therefore subject to a higher risk of damage by the radiation. Therefore suitably, once a detector has measured the reflection from the skin, this measurement may be used in the apparatus according to the invention to automatically calculate and adjust a laser intensity that is convenient for the treatment. However, in case that darker spots on a light skin, for example birthmarks, are to be treated, this function may be omitted.

An applicable laser is a diode laser with a wavelength of 800 nm and a power of 80 mW. The radiation is typically directed onto a spot with a diameter of 0.1 mm. Equipped with such a laser, two commercially available batteries are sufficient for the apparatus to function for more than ten hours.

In order to ease the handling of the laser for depilation, the apparatus may have a laser array for a line focus, for example an array with 1000 mW may be used on a line focus for example of 0.5 mm x 2 mm. Such type of laser may have a very high efficiency and have only small heat production such that active cooling of the apparatus is not necessary.

A laser of this type has been used in test experiments and proved easy handling and efficient hair removal. Irradiation of the skin for as long as minutes has not shown any skin injury.

## Claims

1. Apparatus for laser treatment comprising
- a casing of a size which is easily hand-held,
- a laser system inside said casing including a laser with an electronic circuit, said laser arranged such that produced laser radiation from said laser exits said casing through an exit system of said casing,
- a connection to an electrical power supply to provide power to said apparatus,
**characterised in that** said exit system comprises a safety device that only allows firing of the laser when the direction of said laser radiation is directed onto the surface of the subject to be treated.

2. Apparatus according to claim 1, wherein said safety device comprises at least one from the following
- a mechanical pressure switch arranged to be activated when said exit system of said apparatus is pressed against said surface of the subject to be treated, the pressing being in a direction which requires an orientation of said apparatus such that the laser radiation is directed towards said surface of the subject to be treated;
- a pressure sensor coupled to a safety switch only allowing said laser to fire when the pressure exerted on said pressure sensor is higher than a predetermined level, said pressure being in a direction substantially normal to the surface of the subject to be treated;
- a temperature sensor coupled to a safety switch only allowing said laser to fire when the temperature at the surface of the subject to be treated as measured with said temperature sensor is within a predetermined range of temperatures;
- a pH sensor coupled to a safety switch only allowing said laser to fire when the pH at the surface of the subject to be treated as measured with said pH sensor is within a predetermined range of pH;
- a resistivity sensor coupled to a safety switch only allowing said laser to fire when the resistivity at the surface of the subject to be treated as measured with said resistivity sensor is within a predetermined range of resistivity, for example between 10 kΩ/mm and 1 MΩ/mm;
- a conductivity sensor coupled to a safety switch only allowing the laser to fire when the conductivity at the surface of the subject to be treated as measured with the conductivity sensor is within a predetermined range of conductivity;
- a radiation detector configured to receive reflected laser radiation, said radiation detector being functionally coupled to a safety switch stopping said laser to fire when the intensity of said reflected laser radiation detected by said radiation detector is outside a predetermined range of intensity,
- an additional radiation source, preferably a light emitting diode or an additional laser, and a radiation detector for detection of radiation from said additional radiation source reflected on a surface to be treated, said radiation detector being functionally coupled to a safety switch only allowing said laser to fire when the intensity of said reflected radiation from said additional radiation is within a predetermined range of intensity,
- a radiation detector receiving reflected laser radiation and an evaluation device, preferably an evaluation computer, functionally connected to said radiation detector for determining the type of surface reflecting the laser radiation,
- an additional radiation source, preferably a light emitting diode, and a radiation detector for detection of radiation from said additional radiation source reflected on a surface to be treated, said radiation detector being functionally connected to an evaluation device, preferably an evaluation computer, for determining the type of surface reflecting the laser radiation.

3. Apparatus according to claim 1 or 2, wherein said exit system is configured to create a closed system in combination with the surface of the object to be treated, such that no laser light can escape uncontrolled during use of the apparatus.

4. Apparatus according to any one of the preceding claims, wherein said evaluation device is functionally connected to an intensity adjustment device adjusting the intensity of said laser radiation according to the intensity of the light reflected from said surface.

5. Apparatus according to claim 4, wherein the apparatus is configured to treat the intensity of the reflected radiation as being indicative of the degree of pigmentation of human skin and wherein the intensity of said laser radiation is automatically adjustable in relation to a predetermined relationship between degree of skin pigmentation and intensity of laser radiation.

6. Apparatus according to any one of the preceding claims, wherein said power supply is a battery system inside said casing.

7. Apparatus according to any one of the preceding claims, wherein said laser is a laser diode, preferably with a wavelength of between 600 and 1100 nm, or a laser diode array.

8. Apparatus according to any one of the preceding claims, wherein said apparatus comprises a magnifying lens attached to said casing and directed towards the surface spot for laser treatment, said lens optionally being coated with a film or dyed with a colour such as to prevent or strongly attenuate the transmission of reflected laser light.

9. Apparatus according to any one of the preceding claims, wherein said apparatus comprises an additional switch, said additional switch being arranged resiliently with counter force such that said switch may be pressed but not locked in the pressed state, wherein said laser only can fire when said additional switch is pressed.

10. Apparatus according to any one of the preceding claims, wherein said apparatus comprises a main switch for the main power supply and a main control lamp which is illuminated when said main switch is turned on.

11. Apparatus according to any one of the preceding claims, wherein said apparatus comprises a laser control lamp which is illuminated when said laser is firing, said laser control lamp preferably being a Teflon tip in the exit system, said Teflon tip confining said laser radiation near the treatment spot on said surface.

12. Apparatus according to any one of the preceding claims, wherein said apparatus is provided with a cover for covering said exit system for prohibiting said laser radiation to exit said exit system, said cover being locked to said apparatus with a locking mechanism wherein said cover is only demountable after unlocking of said locking mechanism, said locking mechanism preferably comprising an alphanumerical code lock.

13. Use of an apparatus according to to any one of the preceding claims for laser treatment of hair roots, acne, tattoos, benign pigmented lesions, microvascular malformations, vascular lesions, or wrinkles.

14. A liquid suitable for use for laser treatment by an apparatus according any one of the preceding claims, wherein said liquid has at least one of the group consisting of a preselected marking colour, conductivity, resistivity, pH, or reflectivity, or a cooling effect.

15. A kit for laser treatment comprising an apparatus according any one of the preceding claims and a liquid for application to the surface of the object to be treated, wherein said liquid has at least one from the group consisting of a preselected marking colour, conductivity, resistivity, pH or reflectivity, or a cooling effect.
